# EUROPÄISCHE PATENTSCHRIFT

(11) **EP 3 349 811 B1**
(45) Veröffentlichungstag und Bekanntmachung des Hinweises auf die Patenterteilung: **12.04.2023**
(21) Anmeldenummer: 16767250.0
(22) Anmeldetag: 16.09.2016
(51) Int. Cl.: C07K 5/087, C07K 7/06, C07K 17/14, A61L 27/28, A61L 27/50, A61L 27/54, A61K 47/65, A61K 6/20

(54) **PEPTID ZUR BESCHICHTUNG VON OBERFLÄCHEN**
PEPTIDE FOR COATING SURFACES
PEPTIDE POUR LE REVETEMENT DE SURFACES

(30) Priorität: 17.09.2015 DE 202015006574 U
(43) Veröffentlichungstag der Anmeldung: 25.07.2018
(73) Patentinhaber: Universität Leipzig, 04109 Leipzig (DE)
(72) Erfinder: BECK-SICKINGER, Annette, 04316 Leipzig (DE); PAGEL, Mareen, 08013 Barcelona (ES); HASSERT, Rayk, 04299 Leipzig (DE)
(74) Vertreter: Lederer & Keller Patentanwälte Partnerschaft mbB
(86) Internationale Anmeldenummer: PCT/EP2016/071958
(87) Internationale Veröffentlichungsnummer: WO 2017/046323

(56) Entgegenhaltungen:
- WO-A2-2008/150101
- WO-A2-2008/156637
- US-A1- 2013 052 712
- HASSERT RAYK ET AL: "Tuning peptide affinity for biofunctionalized surfaces", EUROPEAN JOURNAL OF PHARMACEUTICS AND BIOPHARMACEUTICS, Bd. 85, Nr. 1, 27. Februar 2013 (2013-02-27), Seiten 69-77, XP028692958, ISSN: 0939-6411, DOI: 10.1016/J.EJPB.2013.02.006

## Beschreibung

Die vorliegende Erfindung betrifft ein Peptid, eine Beschichtung, ein beschichtetes Implantat, sowie ein Peptid erhältlich durch eine Kombination von Festphasensynthese und Click-Chemie.

Titan (Ti) ist das Material der Wahl bei der Herstellung von Zahnimplantaten und orthopädischen Implantaten. In Folge mangelnder Osseointegration können jedoch Entzündungen, Lockerungen oder Einsinken des Implantats auftreten. Die Osseointegration ist das Ergebnis eines knöchernen Heilungsprozesses, bei dem die Knochenzellen (Osteoblasten) direkt an das Implantat heranwachsen und eine feste Anheftung an der Implantatoberfläche erzielen. Um die Osseointegration des Implantats zu verbessern, wurde versucht die Biokompatibilität der Titanoberfläche mit Hilfe von Komponenten der extrazellulären Matrix (ECM) zu verbessern. Peptidbeschichtungen, welche aus ECM-Komponenten hergestellt wurden spielen eine wichtige Rolle in der Nachahmung von zelladhesiven Eigenschaften. Das bekannteste Peptid ist hierbei das Integrin-Bindepeptid RGD (Arg - Gly - Asp). Neben Integrin-Bindepeptiden wurde eine Bindung an Transmembranproteoglykane für Peptide mit basischen Aminosäuren gezeigt. Es wird angenommen, dass die Peptidsequenz FHRRIKA (Phe - His - Arg - Arg - Ile - Lys - Ala) (SEQ ID NO:1) an Heparin enthaltende Proteoglykane bindet und so die Anlagerung von Zellen unterstützt. Eine direkte Immobilisierung dieser Moleküle ist jedoch meist nur nach einer Funktionalisierung der Oberfläche möglich und benötigt die Integration spezifischer Ankergruppen in dem Peptid. Es ist bekannt, dass L-3,4-dihydroxyphenylalanin (DOPA) an die oxidierte Oberfläche von Titan ohne chemische Behandlung binden kann. Diese posttranlational modifizierte Aminosäure wurde in Proteinen, die von der Miesmuschel (*Mytilus edulis*) abgesondert werden, gefunden.

So beschreibt die WO 2013/183048 A1 Peptide mit einer Titan-bindenden Aminosäuresequenz, die mit einer heterologen Aminosäuresequenz verknüpft ist. Die Titan-bindenden Aminosäuresequenz kann L-DOPA und die heterologe Aminosäuresequenz ein RGD-Motiv enthalten.

Die Druckschrift US 2013/0052712 A1 beschreibt ein Gerüst (*scaffold*), das ein adhäsives Protein aus *Mytilus edulis* und eine bioaktive Substanz enthalten kann. Varianten des Muschelproteins können L-DOPA und ein RGD-Motiv enthalten.

WO 2008/150101 offenbart lineare Fusionsproteine, die auf Grundlage einer Muschelpeptidsequenz mit gentechnischen Methoden hergestellt wurden. Das rekombinante Polypeptid ist mit einem biofunktionale Peptid modifiziert und soll die extrazelluläre Matrix mimikrieren. Unter anderem wird eine Integrin-Bindepeptidsequenz oder eine Heparin-bindende Domäne als Teil des Fusionsproteins genannt.

Die Synthese und insbesondere die spezifische kovalente Modifizierung von rekombinant exprimierten Muscheladhäsionsproteinen mit mehreren bioaktiven Molekülen bleiben schwierig.

Aufgabe der Erfindung ist es daher ein effektives, modifiziertes Molekül bereitzustellen, welches unter anderem die Osseointegration von Implantaten fördert.

Diese Aufgabe wird vorliegend gelöst durch ein Peptid, umfassend (i) eine Hauptkette mit wenigstens einem L-3,4-dihydroxyphenylalanin (DOPA), (ii) wenigstens ein Integrin-Bindepeptid und (iii) wenigstens ein Heparin-Bindepeptid, erhältlich durch eine Kombination von Festphasensynthese und Click-Chemie. Die Kombination von wenigstens einem DOPA, wenigstens einem Integrin-Bindepeptid und) wenigstens einem Heparin-Bindepeptid zeigt eine synergistischen Effekt bei der Zelladhäsion an mit dem Peptid beschichteten Metalloberflächen.

Unter Hauptkette wird vorliegend jegliche Verbindung verstanden, welche Komponenten sowohl peptidischen, als auch nicht-peptidischen Ursprungs enthalten kann, solange die Hauptkette wenigstens ein L-3,4-dihydroxyphenylalanin (DOPA) umfasst. Neben Aminosäuren kann die Hauptkette daher auch nicht-peptidische Komponenten wie Spacer, zum Beispiel Polyethylenglykol(PEG)-Spacer oder Aminohexansäure, enthalten. Bevorzugt umfasst die Hauptkette wenigstens zwei L-3,4-dihydroxyphenylalanin (DOPA). Vorzugsweise besteht die Hauptkette aus mindestens 4 Einheiten, die linear miteinander verknüpft sind, z.B. über Peptidbindungen. In weiter bevorzugten Ausführungsformen besteht die Hauptkette aus wenigstens 5, wenigstens 6, wenigstens 7 oder wenigstens 8 linear verknüpften Einheiten. Ein, zwei oder drei Einheiten sind DOPA. Wenigstens 1, wenigstens 2, wenigstens 3, wenigstens 4, oder wenigstens 5 Einheiten sind üblicherweise Aminosäuren.

Es ist weiterhin bevorzugt, dass wenigstens eine Einheit Lysin ist. Über die ε-Aminogruppe kann das Integrin-Bindepeptid oder das Heparin-Bindepeptid kovalent gebunden sein. Die Hauptkette kann 1, 2 oder 3 Lysine enthalten. Vorzugsweise ist das Integrin-Bindepeptid an ein Lysin gebunden.

Weiterhin enthält die Hauptkette eine Einheit, an die das Integrin-Bindepeptid oder das Heparin-Bindepeptid kovalent gebunden ist. Im erfindungsgemäßen Peptid ist ein möglicher Vorläufer dieser Einheit L-Propargylglycin. Die Propargylgruppe ist im erfindungsgemäßen Peptid nicht mehr enthalten, sondern hat durch die Kopplung reagiert.

Weitere mögliche Bestandteile der Hauptkette oder des erfindungsgemäßen Peptids sind β-Alanin, PEG, Aminohexansäure und Cystein. Die Hauptkette kann am C-Terminus amidiert sein.

In einer Ausführungsform ist die Hauptkette von einem Protein aus der Muschel *Mytilus edulis* abgeleitet. Besonders bevorzugt hat die Hauptkette die Sequenz Cys - PEG - DOPA - Lys - DOPA - PEG - L-Propargylglycin - β-Ala - NH₂ (SEQ ID NO:2), wobei PEG für Polyethylenglykol steht. Ohne an die Theorie gebunden zu sein, dienen die PEG-Spacer einer Erhöhung der Löslichkeit in wässrigen Medien und einer Verminderung der Adhäsion von Proteinen und Bakterien.

Unter Integrin-Bindepeptid wird jedes Peptid verstanden, welches an Integrine binden kann. In einer bevorzugten Ausführungsform umfasst das Integrin-Bindepeptid die Aminosäuresequenz Arg - Gly - Asp (RGD). Besonders bevorzugt ist die cyclische Aminosäuresequenz c[Arg - Gly - Asp - D-Phe - Lys] (cRGDfK).

Unter Heparin-Bindepeptid wird jedes Peptid verstanden, welches an Heparin binden kann. In einer bevorzugten Ausführungsform umfasst das Heparin-Bindepeptid die Aminosäuresequenz Phe - His - Arg - Arg - Ile - Lys - Ala (SEQ ID NO:1).

In einer beispielhaften Ausführungsform hat das Peptid folgende Struktur ("MP-RGD-HBP"):

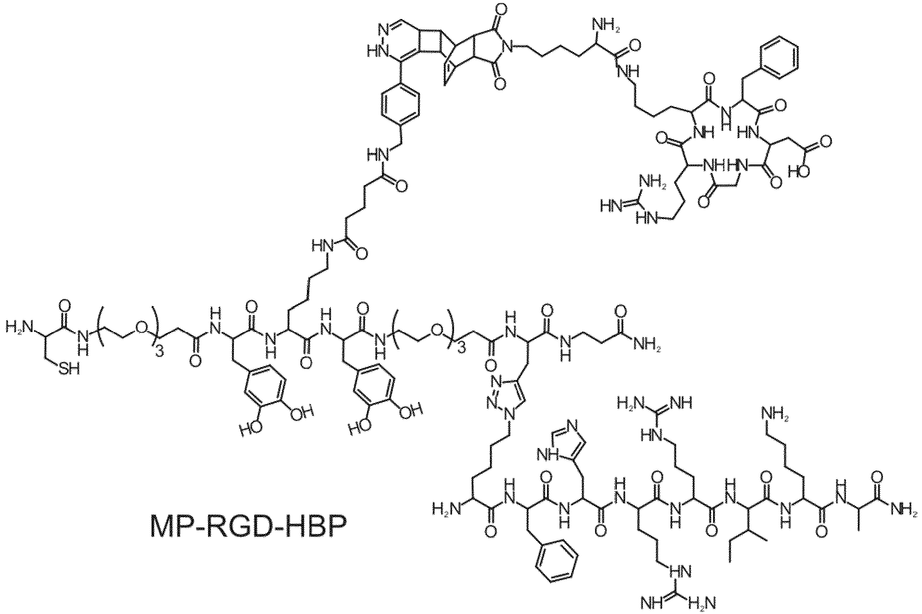

Die oben beschriebenen Peptide binden hervorragend an Metalloberflächen, insbesondere an Titan. So besitzen die Peptide gemäß der vorliegenden Erfindung eine Bindungsaffinität zu Metalloberflächen, bevorzugt zu Titan, bei der die Sättigungsphase im sub-µM-Bereich startet. In einer Ausführungsform liegt die Bindungsaffinität zu Metalloberflächen, bevorzugt zu Titan, im Bereich von 0,1 nM bis 10 µM, bevorzugt im Bereich von 1 nM bis 1 µM und besonders bevorzugt im Bereich von 10 nM bis 100 nM.

Weiterhin besitzen die Peptide eine hervorragende Stabilität. So sind die Peptide der vorliegenden Erfindung bei 37°C über einen Zeitraum von mindestens 1 Woche, bevorzugt mindestens 2 Wochen, besonders bevorzugt mindestens 4 Wochen in wässriger Lösung stabil. Stabil bedeutet hierin, dass über diesen Zeitraum mehr als 50%, bevorzugt mehr als 60%, bevorzugt mehr als 70% des auf der Metalloberfläche immobilisierten und biotinylierten Peptids bestimmt werden kann (Bestimmung wie in den Beispielen durchgeführt, siehe Figur 2C).

Desweiteren betrifft die Erfindung eine Beschichtung für Metalloberflächen, wobei die Beschichtung ein oben beschriebenes Peptid umfasst. Die Beschichtung kann weitere Stoffe, wie zum Beispiel Hilfsstoffe und Lösungsmittel enthalten.

In einer Ausführungsform besteht die zu beschichtende Metalloberfläche aus Titan.

In einer weiteren Ausführungsform wird das zu beschichtende Metall in den menschlichen oder tierischen Körper implantiert. Hierbei kann jegliches Implantat verwendet werden. Bevorzugt ist das Implantat ausgewählt aus der Liste bestehend aus Zahnimplantaten und orthopädischen Implantaten, z.B. Hüftgelenksimplantaten. Desweiteren betrifft die Erfindung eine beschichtete Metalloberfläche, erhältlich durch eine Reaktion von einem oben beschriebenen Peptid mit einer Metalloberfläche. Die Beschichtung kann weitere Stoffe, wie zum Beispiel Hilfsstoffe und Lösungsmittel enthalten.

In einer Ausführungsform besteht die zu beschichtende Metalloberfläche aus Titan.

In einer weiteren Ausführungsform wird das zu beschichtende Metall in den menschlichen oder tierischen Körper implantiert. Hierbei kann jegliches Implantat verwendet werden. Bevorzugt ist das Implantat ausgewählt aus der Liste bestehend aus Zahnimplantaten und orthopädischen Implantaten, z.B. Hüftgelenksimplantaten. Desweiteren betrifft die Erfindung ein beschichtetes Implantat erhältlich durch eine Reaktion von einem oben beschriebenen Peptid mit einem Implantat. Bevorzugt ist das Implantat ausgewählt aus der Liste bestehend aus Zahnimplantaten und orthopädischen Implantaten, z.B. Hüftgelenksimplantaten.

Die vorliegende Erfindung betrifft ein Peptid umfassend (i) eine Hauptkette umfassend wenigstens ein L-3,4-dihydroxyphenylalanin (DOPA), (ii) wenigstens ein Integrin-Bindepeptid und (iii) wenigstens ein Heparin-Bindepeptid, erhältlich durch eine Kombination von Festphasensynthese und Click-Chemie.

Unter Festphasensynthese wird hierin die chemische Synthese von Stoffen verstanden, die während der Synthese über kovalent verknüpfende Gruppen (sog. Linker) an feste Phasen gekoppelt sind, um nach erfolgter Synthese durch Abspaltung der Linkergruppen freigesetzt zu werden. Beispielsweise erfolgt die Festphasensynthese unter Anwendung der Fluorenylmethoxycarbonyl / tert-butyl (Fmoc/tBu) Strategie.

Unter Click Chemie wird vorliegend das von K. Barry Sharpless mit Hartmuth C. Kolb und M. G. Finn begründete Konzept verstanden, welches es ermöglicht schnell und zielgerichtet Zielmoleküle aus kleineren Einheiten zu synthetisieren (Kolb et al., Angew. Chemie 2001, Band 113, S. 2056). Bevorzugt handelt es sich hierbei um Zykloadditionsreaktionen, wie zum Beispiel die Cu(I) katalysierte Huisgenzykloaddition (Azid-Alkin Zykloaddition) und die Diels-Alder-Reaktion mit inversem Elektronenbedarf.

### Beschreibung der Figuren

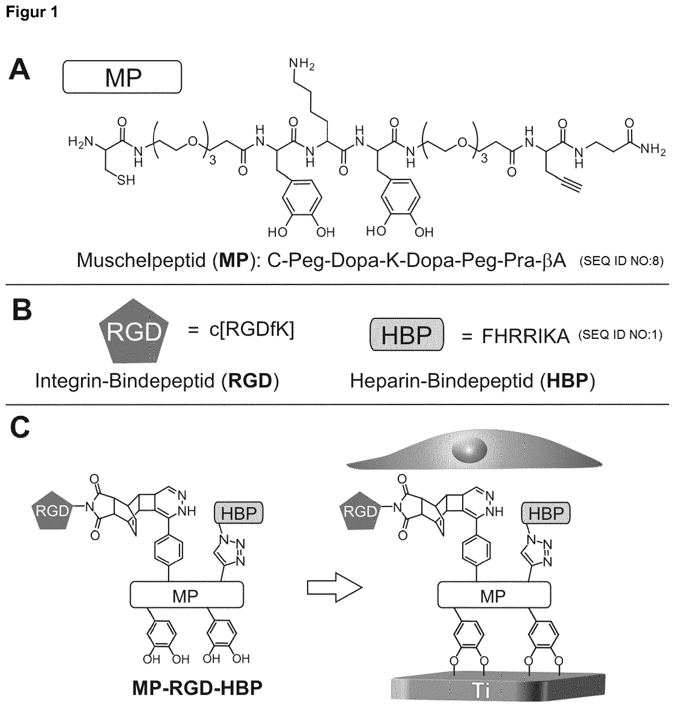

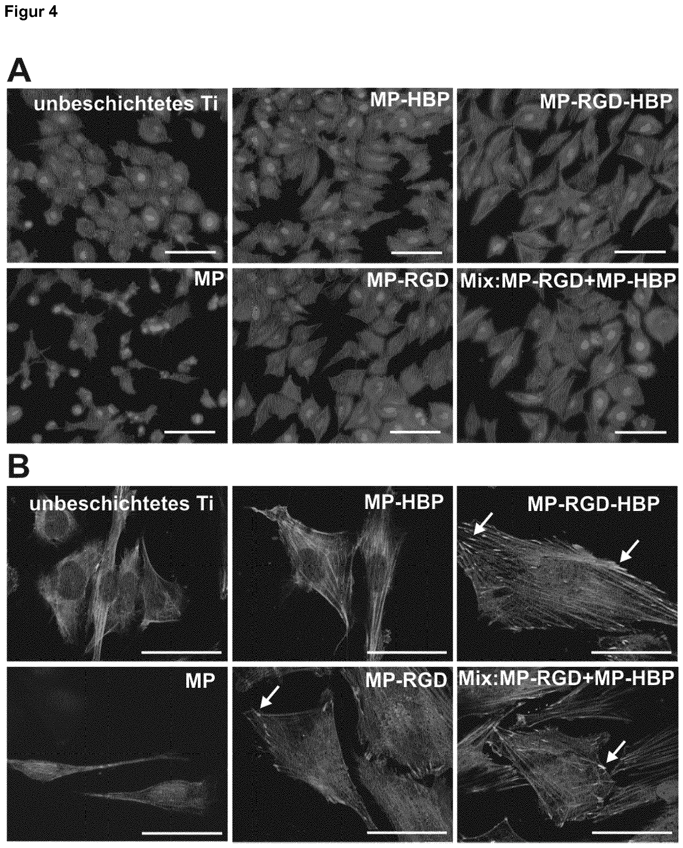

**Figur 1****:** Design eines multifunktionalen Peptids
   (A) Sequenz des Titan-adhäsiven Muschelpeptids (MP); Das Peptid besteht aus der Titanbindenden Aminosäure (L-3,4-Dihydroxyphenylalanin), funktionellen Gruppen eingeführt durch Pra (L-Propargylglycin), Cys and Lys als auch aus spacer (Abstandshalter) wie PEG (Polyethylenglycol) und β-Ala. (B) Zellbindepeptide: zyklisches Integrin-Bindepeptid (RGD), lineares Heparin Bindepeptid (HBP), (C) Ligationsprodukt bestehend aus MP, RGD und HBP, synthetisiert mithilfe von orthogonalen Zykloadditionen (CuAAC and DARᵢₙᵥ), und die daraus resultierende Anwendung eines "Zell-Titan-Klebers".

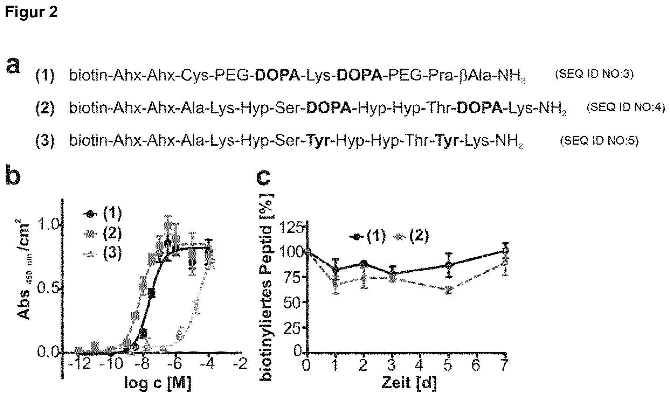

**Figur 2****:** Bindungseigenschaften und Stabilität von MP auf Titan
   (A) Sequenz des natürlich abgeleiteten (mefp-1) und des artifiziellen Peptids (MP); Hyp (4-Hydroxyprolin), Ahx (Aminohexansäure). (B) Konzentrationswirkungskurve des Biotin-ELISA in der die im sub-µM Bereich startende Sättigungsphase für MP und mefp-1 zu erkennen ist. (C) Stabilität der DOPA-Peptide auf Titan, inkubiert mit Zellüberstand bei 37° (bestimmt durch einen Biotin-ELISA).

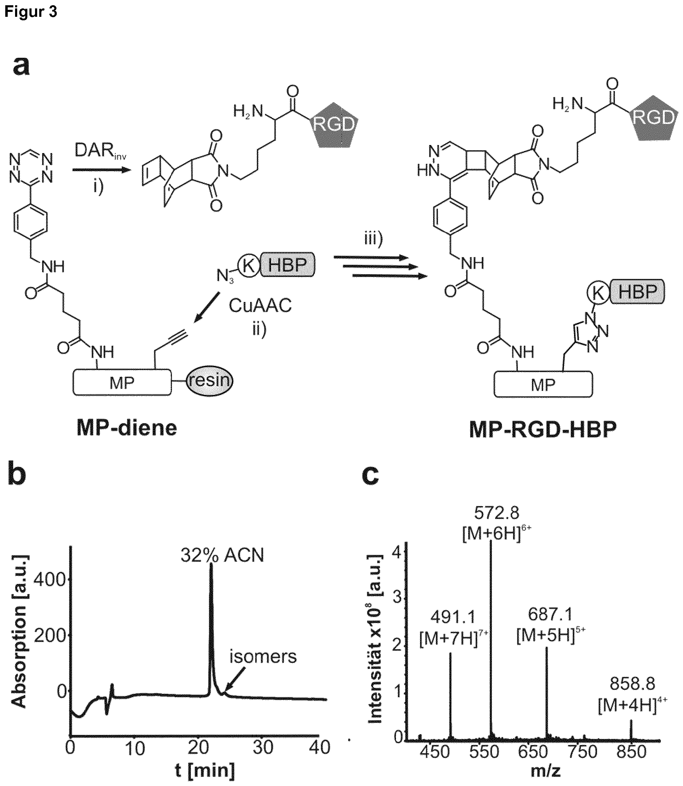

**Figur 3****:** Orthogonale Zykloadditionen am Harz um das multifunktionale Peptid MP-RGD-HBP zu erhalten
   (A) Reaktionsschema der DARᵢₙᵥ und CuAAC; Reagenzien und Bedingungen: a) Wasser, RT, 5h; b) CuSO₄, THPTA, TCEP in Wasser, RT, 1 d; nach Inkubation mit einer wässrigen EDTA-Lösung wurde das Peptid mit scavenger und TFA vom Harz abgespalten (B) RP-HPLC Chromatogramm und (C) ESI-Ionenfalle-MS des gereinigten MP-RGD-HBP
**Figur 4****:** Zelladhäsion auf Peptidbeschichtungen in Medium ohne FCS (fetales Kälberserum)
   Aktinzytoskelett in rot, Zellkern in blau und Vinkulin in grün (A) Fluoreszenzmikroskopie von SaOS-2 Zellen nach 6 h Adhäsion auf synthetisierten Peptiden und der Negativkontrolle (unbeschichtetes Ti), Maßstab: 100 µm (B) Immunmarkierung von fokalen Kontakten: Fluoreszenzmikroskopiebilder von SaOS-2 Zellen nach 24 h Adhäsion auf Peptidbeschichtungen, Maßstab bar: 50 µm.

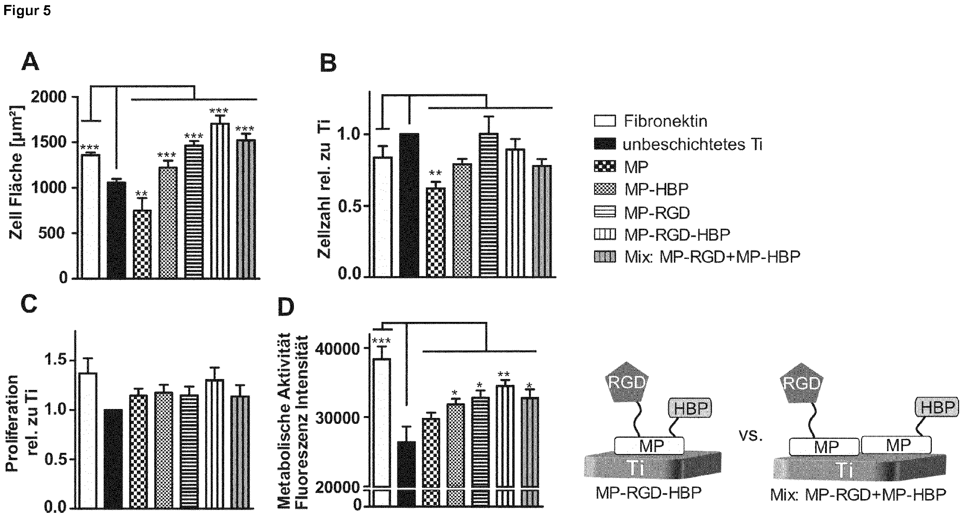

**Figur 5****:** Zellantwort auf Peptidbeschichtungen
   A) durchschnittliche Zellfläche und B) Zellzahl normalisiert zu unbeschichteten Ti= 1 nach 6 h Adhäsion in Serum freien Medium, ermittelt durch Fluoreszenzmikroskopie. C) Proliferation (BrdU-assay, 24h Inkorporation) normalisiert auf Ti= 1 und D) Viabilität (Resazurin assay) nach 3 Tagen Zelladhäsion (24h in Serum freien Medium, gefolgt von FCS-haltigen Medium). Ergebnisse werden als Mittel ± SEM aus n ≥3 (signifikante Unterschiede zu unbeschichtetem Titan wurden durch one-way ANOVA (***p< 0.001) ermittelt) Schematische Darstellung: multifunktionales Peptid *versus* Mischung einfachmodifizierter Peptide.

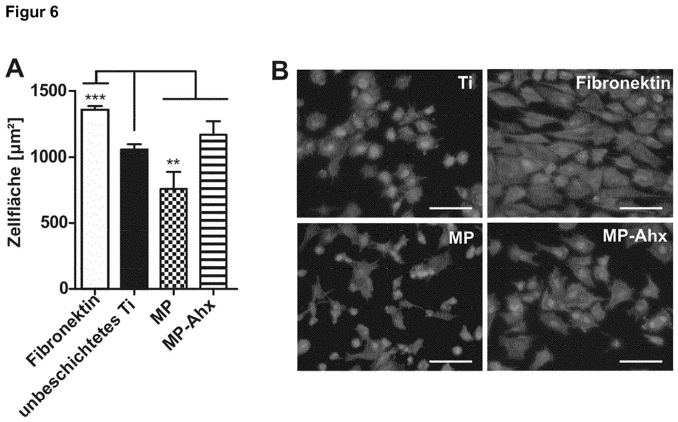

**Figur 6****:** Zellausbreitung auf Peptibeschichtungen (A) Durchschnittliche Zellfläche (Standartfehler des Mittels. n= 3) (B) Fluoreszenzmikroskopie von SaOS-2 Zellen nach 6h Adhäsion auf den Peptidbeschichtungen. Das Aktinzytoskelett ist in rot angefärbt und der Zellkern in blau Maßstab: 100µm.

### Beispiele

### Titan-Bindepeptid (MP)

Um ein multifunktionales Titan-Bindepeptid zu erhalten wurde ein von der Muschel abgeleitetes Peptid entworfen, welches funktionale Gruppen enthält (Figur 1A). Zudem wurde ein PEG-spacer eingeführt der zum einen folgende Zykloadditonsreaktionen erleichtern soll und zum anderen die wässrige Löslichkeit erhöht sowie die Adhäsion von Proteinen und Bakterien vermindert.^{1,2}

Im Anschluss an die Synthese des Muschelpeptids durch Festphasenpeptidsynthese (Fmoc/ tBu Strategie) wurde die Bindungsaffinität zu Titan untersucht. Um gebundenes Peptid mithilfe eines bereits beschriebenen Biotin-ELISAs zu untersuchen, wurden die verwendeten Peptide N-terminal biotinyliert (zwei Aminohexansäuren wurden als Abstandshalter eingeführt).³ Als Positivkontrolle wurde, bio-(+) mefp-1, abgeleitet von der natürlich vorkommenden Muschelfußproteinsequenz mefp-1 *(M. edulis* Fuß Protein-1), genutzt um die Affinität zu MP einschätzen zu können (Figur 2A).⁴ Als Negativkontrolle wurde die Aminosäure DOPA durch Tyrosin ausgetauscht (bio-(-) mefp-1) um die Catechol-vermittelte Binding an Titan nachzuweisen (Figur 1C).

Dazu wurden Titanplättchen angeätzt um eine frisch oxidierte Oberfläche zu erlangen. Anschließend wurden die Titanplättchen mit verschiedenen Peptidkonzentrationen inkubiert. Nach intensiven Waschschritten wurde an Titan gebundenes Peptid mit der gut beschriebenen Biotin-Streptavidin Interaktion nachgewiesen. Peptide, die DOPA enthalten, zeigten hohe Bindungsaffinitäten mit einer in sub-µM-Bereich starteten Sättigungsphase (Figur 2B). Das artifiziell entworfene Peptid MP (EC₅₀ = 23.6 nM, pEC₅₀ = 7.6 ± 0.1) bindet dabei mit ähnlicher Affinität wie das natürlich abgeleitete mefp-1- Peptid (EC₅₀ = 6.4 nM, pEC₅₀ = 8.2 ± 0.1). Dramatisch verringerte Adhäsionseigenschaften wurden durch das Tyrosin-Peptid bio-(-) mefp-1 (EC₅₀ > 10 000 nM, pEC₅₀ = 4.6 ± 0.1) ermittelt. Darüber hinaus wurde die Stabilität der DOPA-Peptide in Zellüberstand getestet um *in vitro* Bedingungen zu imitieren. Dafür wurden Titanplättchen mit den entsprechenden biotinylierten Peptiden (Konzentration = 1 µM) beschichtet. Nach Inkubation in SaOS-2 Zellüberstand bei 37°C wurde verbleibendes Peptid mithilfe des Biotin-ELISAs nachgewiesen. Im Zeitraum von 7 Tagen wurde konstant mehr als 50% immobilisiertes und biotinyliertes Peptid bestimmt (Figur 2C). Zudem konnten keine signifikanten Unterschiede zwischen artifiziellen(bio-MP) und natürlich abgeleiteten Peptid (bio-(+) mefp-1) festgestellt werden. Jedoch zeigte MP eine Tendenz zur höheren Stabilität.

### Das Einführen von Multifunktionalität-Bioorthogonale Zykloadditionen an fester Phase

Selektive Zelladhäsion auf anorganischen Oberflächen kann durch verschiedene zellbindende Motive vermittelt werden. Durch Konjugation dieser Motive mit titanbindenden Peptiden entsteht ein Zell-Oberflächenmediator. Jedoch ist die Ligation komplexer Peptide, wie dem zyklischen αᵥβ₃ selektiven Liganden c[RGDfK] besonders anspruchsvoll. Daher entwickelten wir eine mit der Festphasenpeptidsynthese kompatiblen Methode, die auf der Diels-Alder Reaktion mit inversem Elektronenbedarf (DARᵢₙᵥ) (eine Reaktion zwischen einem Dien und Dienophil) beruht. Daher wurde das RGD-Peptid an der Lys-Seitenkette mit einem Dienophil modifiziert (Reppe Anhydrid-Lysin-Derivat). Dementsprechend wurde MP mit einem Tetrazin, welches das Dien darstellt, modifiziert. Um die DARᵢₙᵥ durchzuführen wurde MP-dien, am Harz gebunden, mit einer wässrigen Lösung des c[RGDfK(dienophil)] für 5h inkubiert. Das erwünschte Konjugat MP-RGD wurde dann von der festen Phase abgespalten und konnte mithilfe von MALDI-ToF-MS und RP-HPLC identifiziert werden. Neben der DARᵢₙᵥ wurde die orthogonale CuAAC genutzt um ein Heparin-Bindepeptid an MP-RGD zu ligieren. Vorrausgehend dazu wurde das MP mit einer Alkingruppe, durch Propargylglycin eingeführt, am C-Terminus modifiziert. Demzufolge wurde Azido-Lysin an HBP gekuppelt und darauffolgend vom Harz abgespalten. Das aufgereinigte Peptid HBP-N₃ wurde dann genutzt um die CuAAC mit am Harz gebundenen MP-RGD durchzuführen. Abschließende Abspaltung des ligierten Peptides zeigte dass sich das gewünschte Produkt mit einem Umsatz von 75% gebildet hat. Nach Aufreinigung wurden das Produkt und seine entsprechenden Isomere mit RP-HPLC sowie ESI-MS analysiert (Figur 3C).

### Zelladhäsion auf Peptid-beschichtetem Titan

Um die Bioaktivität der ligierten Peptide sowie deren Kontrollen zu bestimmen wurden Zelladhäsionsstudien durchgeführt. Osteoblasten-ähnliche Zellen (SaOS-2) wurden auf angeätzten und mit Peptid beschichteten Titan ausgesät. Alle zellulären Experimente wurden in Serum-freiem Medium durchgeführt um falsche Ergebnisse durch die Adsorption von Serumproteinen zu verhindern. Um die initiale Zelladhäsion zu untersuchen wurden die Zellen nach 6h Kultur fixiert und gefärbt um die Morphologie sowie die durchschnittliche Zellfläche-und Zahl mithilfe von Fluoreszenzmikroskopie zu beobachten. Dabei wurde die geringste Zellausbreitung, auf MP beschichtetem Titan ermittelt, signifikant zu unbeschichtetem Titan. Zudem zeigten die Zellen eine eher runde Form mit schwach ausgebildeten Zytosekelett induziert durch die Zellabweisenden Eigenschaften von PEG.⁵ Um dies genauer zu untersuchen wurde PEG durch Aminohexansäure ausgetauscht. Tatsächlich wurde ein Anstieg der Zellgröße beobachtet, etwas höher als auf unbeschichteten Titanplättchen. Die Modifizierung von MP mit HBP resultierte in einem Anstieg der Zellausbreitung im Vergleich zu MP und unbeschichtetem Titan. Jedoch bildeten die Zellen nur wenig Aktinfilamente aus und adhärierten in unregelmäßigen Zellformen (Figur 5A). Noch bessere Resultate konnten durch eine MP-RGD-Beschichtung erlangt werden, da eine durchschnittliche Zellgröße vergleichbar mit dem natürlich vorkommenden ECM-Protein Fibronektin festgestellt werden konnte. Der Austausch von RGD zu RAD führte zu einer Verringerung der Zellgröße mit einer Zellfläche etwas höher als auf unbeschichtetem Titan. Die Kombination aus HBP und RGD in einem Peptid induzierte die höchste durchschnittliche Zellfläche, signifikant zu beiden Kontrollen (Titan, Fibronektin), MP, MP-HBP und MP-RGD. Andererseits führte eine Mischung aus MP-HBP und MP-RGD zu keinem synergistischen Effekt, da eine Zellausbreitung vergleichbar zu MP-RGD ermittelt werden konnte (Figur 4 und 5A). Zellen mit einer Fibroblast-ähnlichen Morphologie und ausgeprägten Fibrillenbündel wurden auf allen RGD-haltigen Peptidbeschichtungen gefunden. Besonders auf MP-RGD-HBP bildeten sich stark ausgeprägte, parallel verlaufende Aktinfilamente aus (Figur 4). Neben der durchschnittlichen Zellfläche wurde die Zellzahl durch Zählen der nach 6h adhärierten Zellen bestimmt. Dabei vermittelte MP aufgrund des zellabweisenden Effekts des PEGs die kleineste Zellzahl, bemerkenswerterweise signifikant weniger als auf unbeschichteten Titan (Figur 5B). Darüber hinaus konnte eine ähnliche Tendenz wie schon im Zellflächen-Experiment beobachtet werden. Dabei wurde die Zellzahl auf RGD zu einem größer Ausmaß als auf HBP erhöht. Zudem führte eine Mischung aus RGD und HBP zu einem kleineren Effekt als RGD und HBP kombiniert in einem Peptid. Es ist bekannt dass RGD-Peptide die Bildung von fokalen Kontakten, die für die Außen-Innen Kommunikation zwischen Zelle und ECM entscheiden sind, vermitteln.⁶ Durch Immunmarkierung mit einem Anti-Vinkulin Antikörper ist es möglich Vinkulin, ein Protein das im Aufbau von Fokaler Adhäsion involviert ist, visualisierbar zu machen. Fluoreszenzmikroskopiebilder zeigten in der Tat, dass Strich-ähnliche Adhäsionskontakte (in grün) auf MP-RGD, MP-RGD-HBP sowie auf Mix: MP-HBP+RGD gebildet wurden, hauptsächlich an der Peripherie der Zelle (Figur 4B). Eine leichte Zunahme an fokalen Komplexen konnte auf MP-RGD-HBP verglichen zu einfach modifizierten RGD-Beschichtungen beobachtet werden. Im Gegensatz dazu führten MP, MP-HBP und unbeschichtetes Titan zur Bildung von wenig oder keinen fokalen Adhäsionskontakten.

Neben initialer Zelladhäsion nach 6h, wurde die Zellviabilität und Proliferation nach 3d untersucht. Der Einbau des Brom-markierten Thymidin Analogs Bromdesoxyuridin (BrdU) in einem Zeitraum von 24h, nach 2d Zelladhäsion, wurde genutzt um die Proliferation der Zellen auf beschichteten Titan zu bestimmen. Keine signifikanten Veränderungen konnten zwischen den immobilisierten Peptiden und Kontrollen festgestellt werden (Figur 5C). Jedoch zeigt sich eine Peptidbeschichtung vorteilhaft gegenüber unbehandeltem Titan. Zudem konnte eine leicht erhöhte Zellproliferation auf MP-RGD-HBP ermittelt werden. Zellviabilität wurde nach 3 Tagen Adhäsion mittels Umsetzung von Resazurin zu Resorufin durch lebende Zellen untersucht. Alle bioaktivmodifizierten Peptidbeschichtungen weisen eine signifikant verbesserte Zellviabilität im Vergleich zu unbeschichtetem Titan auf. Obwohl MP die niedrigste Zellgröße und -Zahl vermittelte, konnten auf dieser Beschichtung mehr lebende Zellen als auf Titan analysiert werden. Dieser Fakt unterstreicht die Zell- abstoßenden aber gleichzeitig nicht toxischen Eigenschaften des PEG. Insbesondere MP-RGD-HBP führt zum besten Ergebnis unter allen synthetisierten Peptiden. MP-RGD induziert eine ähnliche Zellviabilität wie eine Mischung aus MP-RGD und MP-HBP. Damit wird der vorteilhafte Ansatz beide Zell-bindenden Peptide in einem Molekül zu präsentieren, bestätigt.

### Experimenteller Teil

### Materialien und Methoden

Fmoc geschützte Aminosäuren und Reagenzien für die Peptidsynthese wurden von Novabiochem, IrisBiotech, Sigma Aldrich, Roth, Bachem und Orpegen OPC erhalten. Lösungsmittel wurden von Biosolve und VWR bezogen. Das Dienophil Reppe-Anhydrid-Lysin-Derivat wurde wie an anderer Stelle beschrieben, synthetisiert.⁷ Das Tetrazin 5-[4-(1,2,4,5-Tetrazin-3-yl)benzyl- amin]-5-oxopentansäure wurde von Sigma Aldrich bezogen. Reagenzien für die Kupfer (I) katalysierte Azid-Alkin Zykloaddition (CuAAC) wurden von Sigma Aldrich bereitgestellt. Zellkulturmaterial wurde von PAA, Lonza und Biochrome erhalten.

### Synthese des Heparin-Bindepeptids (HBP) und mefp-1 Peptidvarianten

HBP sowie mefp-1- (*Mytilus edulis* Fußprotein-1) abgeleitete Peptide wurden mithilfe von automatisierter Festphasenpeptidsynthese (Syro peptide synthesizer, MultiSynTech) an Rink- Amid Harz (15 µmol, loading 0.7 mmol/g) mit Standard Fluorenylmethoxycarbonyl / tert-butyl (Fmoc/tBu) Strategie hergestellt. Ein detailliertes Protokoll ist von Böhme et al. beschrieben.⁸ Die unnatürliche Aminosäure Fmoc-Lys(N₃)-OH wurde manuell an den N-Terminus von HBP mit 2 eq 1-Hydroxybenzotriazol (HOBt) und diisopropylcarbodiimid (DIC) in Dimethylformamid (DMF) für 4 h gekuppelt. Peptide, die für den Biotin ELISA genutzt wurden, wurden N-terminal mit zwei Einheiten Fmoc-Ahx-OH und schlussendlich mit Biotin durch HOBt/DIC Aktivierung modifiziert.

### Synthese des Muschelpeptids (MP)

Muschel abgeleitete Peptide wurden manuell an TentaGel S RAM (IrisBiotech) synthetisiert. Entschützung der α-Amino-Gruppe wurde zweimal mit 30 % (v/v) Piperidin in DMF für 10 min ausgeführt. Anschließend wurden folgende Aminosäuren equimolar mit 2 eq HOBt/DIC in DMF gekuppelt: Fmoc-β-Ala-OH, Fmoc-Pra-OH, Fmoc-DOPA(Acetonid)OH, Fmoc-Lys(Mtt)-OH, Fmoc-Ahx-OH, Fmoc-Cys(Trt)-OH. Für die Aktivierung des Aminosäurederivats Fmoc-NH-(PEG)₂-COOH (13 atoms) wurden 1.5 eq O-(7-Azabenzotriazol-1-yl)-N,N,N',N'-tetramethyluronium-hexafluorphosphat (HATU) und equimolare Mengen an Diisopropylethylamin (DIPEA) verwendet. Selektive Abspaltung der Schutzgruppe 4- Methyltrityl (Mtt) wurde mit 2 % Trifluoressigsäure (TFA), 3 % Triisopropylsilan (TIS) in Dichlormethan (DCM) (v/v/v) durchgeführt. Anschließend wurde das Dien 5-[4-(1,2,4,5-Tetrazin-3-yl)benzyl- amino]-5-oxopentansäure in zweifachen Überschuss mit HOBt/DIC für 16 h gekuppelt. Biotinylierung des MP wurde wie bereits beschrieben durchgeführt.

### Synthese des c[RGDfK(Dienophil)]

Das zyklische RGD-Peptid wurde wie beschrieben in Hassert et al. synthetisiert.³

### Diels-Alder Reaktion mit inversem Elektronenbedarf (DARᵢₙᵥ) an der festen Phase

TentaGel Harz beladen mit dem Dien-modifizierten Muschelpeptid wurde in Wasser gequollen. Anschließend wurde das Harz mit einer wässrigen Lösung des c[RGDfK(Dienophil) (1.5 eq, 30 mM) für 5 h bei Raumtemperatur auf einem Schüttler in einem offenen Reaktionsgefäß (N₂ Freisetzung) inkubiert. Nach Waschschritten mit Wasser, DMF und DCM, wurde Fmoc N-terminal abgespalten. Schlussendlich wurde das Peptid vom Harz abgespalten.

### Kupfer (I) katalysierte Azid-Alkin Zykloaddition (CuAAC) an fester Phase

TentaGel Harz beladen mit MP beziehungsweise MP-RGD wurde in Wasser gequollen. Für die Modifizierung von MP, wurden 2 eq CuSO₄·5 H₂O (10 mM), 4 eq Tris(2-carboxyethyl)phosphin (TCEP, 0.1 M) und 0.2 eq Tris[(1-benzyl-1H-1,2,3-triazol-4-yl)methyl]amin) (TBTA) in H₂O/ acetonitril (ACN) (3:2, 2 mM) gemischt, entgast mit Ar und für 15 min bei 37 °C inkubiert. Anschließend wurde 1 eq. des Peptids HBP-N₃ zu der Mischung hinzugefügt und der pH-Wert auf pH 8 mit einer wässrigen NaOH-Lösung (10 mM) eingestellt. Nachdem das Harz mit der Reaktionsmischung inkubiert wurde, wurde alles erneut mit Ar entgast und für 44 h bei 37 °C geschüttelt. Für die Modifizierung von MP-RGD, wurde eine Mischung aus 4 eq. CuSO₄·5 H₂O (0.1 M), 8 eq. TCEP (0.1 M) und 6 eq Tris(3-hydroxypropyltriazolylmethyl) amine (THPTA) in H₂O (50 mM) entgast und wie beschrieben für MP behandelt. Dann wurden 1.5 eq HBP-N₃ (10 mM) hinzugefügt und die Mischung wurde auf pH9 eingestellt. Nachdem das Harz mit der Reaktionsmischung inkubiert wurde, wurde entgast und für 24h bei RT geschüttelt. Nach Filtration und Waschschritten wurde das Harz mit einer wässrigen Ethylendiamintetraessigsäure (EDTA, 10 mM) Lösung inkubiert um Kupferrückstände zu entfernen.

### Abspaltung der Peptide vom Harz

Voll- und Probeabspaltungen der Peptide wurde mit einem TFA/scavenger (90:10 v/v) Gemisch unter Schütteln bei RT für 3h durchgeführt. Aufreinigung der Peptide wurde mithilfe von präparativer Umkehrphasen Hochleistungsflüssigkeitschromatographie (RP-HPLC) ausgeführt. Peptide wurden mit Matrix-unterstützter Laser- lonisation-Flugzeitanalysator (MALDI-ToF)-Massenspektrometrie (MS) (Bruker Daltonics) sowie Elektrospray-Ionisations (ESI)-Ionenfallen MS (Bruker Daltonics) identifiziert. Die Reinheit wurde mit RP-HPLC analysiert. Gemessene m/z- Werte stimmten mit den kalkulierten Molekulargewichten überein. Die hergestellten Peptide wurden schlussendlich mit einer Reinheit ≥90% erhalten.

### Biotin-Enzyme verbundener Immunosorbent Assay (ELISA)

Titanfolie (Sigma Aldrich, Dicke: 0.127 mm) wurde in runde Stücke (Fläche= 0.43 cm²) geschnitten und in H₂SO₄ (30 %)/ H₂O₂ (1:1 v/v) für 7 min angeätzt. Nach Waschschritten mit Wasser und TBS Puffer (50 mM Tris(hydroxymethyl)-aminomethan (Tris), 150 mM NaCl, pH 7.6), wurden die Titanplättchen mit verschiedenen Konzentrationen an biotinylierten Peptiden in TBS-Puffer gelöst für 2 h bei RT inkubiert. Anschließend wurden die beschichten Plättchen 5-mal mit TBS-T Puffer (50 mM Tris, 150 mM NaCl, 0.1 % Tween 20, pH 7.6) gewaschen und in neue Reaktionsgefäße überführt. Der folgende Biotin ELISA wurde entsprechend zu Hassert et al. durchgeführt.³ Nullwerte wurden mit nicht beschichteten Titanplättchen erhalten und von den generierten Werten abgezogen (Absorption per cm²). Die erhaltenen Datenpunkte wurden nicht-linear mit GraphPad Prism 5 software (GraphPadlnc.) gefittet. Fehlerbalken sind als Standartfehler des Mittels (SEM) aus mindestens 2 unabhängigen Experimenten dargestellt.

### Stabilitätstest mithilfe des Biotin ELISA

Titanplättchen wurden wie beschrieben geätzt und mit 1 µM Peptidlösungen in TBS-Puffer über Nacht bei RT inkubiert. Anschließend wurden die beschichteten Plättchen 5-mal mit TBS-T Puffer gewaschen und in neue Reaktionsgefäße überführt. Danach wurden die Titanplättchen mit Zellüberstand (von SaOS-2 Zellen nach 2 Tagen Zellkultur) bei 37 °C für bestimmte Zeitpunkte inkubiert. Um gebundenes, biotinyliertes Peptid an der Oberfläche zu detektieren, wurden die Titanplättchen 4-mal mit TBS-T und einmal mit TBS gewaschen und wie beschrieben wurde der Biotin ELISA durchgeführt. Nullwerte wurden durch unbeschichtete Titanplättchen erhalten und von den generierten Daten abgezogen. Die erhaltenen Messwerte wurden zum bei 0 min gemessenen Wert normalisiert. Fehlerbalken sind als Standartfehler des Mittels (SEM) aus mindestens 2 unabhängigen Experimenten (Zweifachbestimmung) dargestellt.

### Zellkultur

SaOS-2 Zellen (osteogenes Sarcoma), wurden in humider Atmosphäre bei 37 °C und 5 % CO₂ in McCoy's 5A Medium mit 15 % hitzeinaktiviertem fetalem Kälberserum (FCS), 1 % (v/v) Glutamin und 1 % (v/v) Penicillin/ Streptomycin kultiviert.

### Präparation der Titanplättchen für zelluläre Assays

Titanfolie wurde gestanzt und geätzt wie bereits beschrieben. Nach Waschritten mit Wasser und PBS-Puffer (Dulbeccos Phosphat), wurden die Titanplättchen mit 70 % Ethanol/ Wasser (v/v) in einem Ultraschallbad für 15 min sterilisiert. Nach zweimal Waschen mit PBS-Puffer wurden die Titanplättchen über Nacht in einer 48-well Platte bei RT mit 300 µL einer 1 µM Peptidlösung in PBS, PBS (Negativkontrolle) und Fibronektin in PBS (25 µg/mL, Positivkontrolle) inkubiert.

### Zelladhäsions-Assay

Beschichtete Titanplättchen (48-well Platte) wurden zweimal mit PBS gewaschen. SaOS-2 Zellen wurden in Medium ohne FCS resuspendiert. Danach wurden die Zellen auf den beschichteten Titanplättchen ausgesät (50,000-90,000 Zellen pro well, in diesem Bereich haben wir keinen Einfluss auf die Zellfläche für die jeweilige Beschichtung festgestellt). Nach der Adhäsion für 6h wurden die Plättchen mit PBS gewaschen und die Zellen wurden mit 4 % Paraformaldehyd in PBS für 30 min fixiert. Die adhärierten Zellen wurden anschließend gewaschen und mit 1 % Triton X-100 in PBS für 1 min permeabilisert. Danach wurden die Zellen mit Phalloidin-Tetramethylrhodamineisothiocyanate (phalloidin-TRITC) und HOECHST 33342 (beide Sigma Aldrich) angefärbt. Für die Fluoreszenzmikroskopie (Axio Observer microscope, Zeiss) wurden die Plättchen auf Objektträger befestigt. Für jedes Plättchen (Triplikat) wurden 3 repräsentative Bilder bei 20- facher und 10- facher Vergrößerung aufgenommen. Die durchschnittliche Zellfläche wurde dann durch Umranden (Software Axio Vision 4.8, Zeiss) jeder einzelnen Zelle auf je einem repräsentativen Bild (20fach) eines jeden Triplikats bestimmt. Die ermittelten Werte wurden aus 3 unabhängigen Experimenten zusammengefasst und als Mittel ± SEM dargestellt. Um die Zellzahl zu bestimmen, wurden drei 10-fach vergrößerte Bilder mithilfe der Software ImageJ ausgewertet und auf Titan=1 normalisiert, n≥ 3.

### Immunomarkeriung

Zellen (40,000 pro well) wurden auf beschichteten und gewaschenen Titanplättchen wie bereits beschrieben ausgesät. Nach 1 d Zelladhäsion wurden die Plättchen zweimal mit PBS gewaschen. Alle folgenden Waschschritte wurden mit 0.05 % (v/v) Tween-20 in PBS durchgeführt. Danach wurden die Zellen fixiert und permeabilisiert, wie bereits beschrieben. Zur Blockierung unspezifischer Interaktionen wurden die Plättchen mit 1 % bovinem Serumalbumin (BSA) in PBS für 40 min inkubiert. Anschließend wurden die Zellen mit einem monoklonalen Maus-Antikörper gegen Vinkulin (Millipore, staining kit, 1: 200) für 1 h bei RT inkubiert. Danach wurden die Zellen gewaschen und mit Phalloidin-TRITC und einem Fluoresceinisothiocyanate (FITC) - konjugierten Ziege Anti-Maus Antikörper (Millipore, 1:100) in 1 % (v/v) BSA in PBS für 2 h markiert. Nach einem weiteren Waschschritt wurde der Zellkern mit 4',6-Diamidino-2-phenylindol (DAPI, Millipore) in PBS für 15 min angefärbt. Bevor die Plättchen auf Objektträgern befestigt wurden, wurden sie dreimal für je 10 min gewaschen. Fluoreszenzmikroskopie wurde an einem Axio Observer Mikroskop und einem ApoTome Imaging System (Zeiss) durchgeführt.

### Viabilitäts Assay

Zellen wurden auf beschichteten und gewaschenen Titanplättchen in FCS-freien Medium in einer 96-well Platte ausgesät. Nach einem Tag wurde das Medium zu FCS-haltigen Medium ausgetauscht. An Tag 3 wurden 20 µl CellTiderBlue^{®} (Promega) zu jedem well hinzugefügt. Nach 2 h Inkubationszeit wurde die Umsetzung von Resazurin zu Resorufin mittels Fluoreszenzintensitätsmessung bei 560Ex/590Em nm bestimmt. Die Messwerte (Mittel± SEM) wurden aus 4 unabhängigen Experimenten, in Triplikaten durchgeführt, ausgewertet.

### Proliferations Assay

Zellen wurden auf beschichteten und gewaschenen Titanplättchen in FCS-freien Medium in einer 96-well Platte ausgesät. Nach einem Tag wurde das Medium zu FCS-haltigen Medium ausgetauscht. An Tag 2, wurde zur Markierung Bromodeoxyuridin (BrdU) zu jedem Well hinzugefügt (colorimetric Cell Proliferation kit, Roche). Nach 24 h Inkorporation des labels wurden die Zellen gewaschen und fixiert. Anschließend wurde mit einem Peroxidase (POD) -konjugierten anti-BrdU Antikörper inkubiert. Das Binden des Antikörpers wurde mit der Zugabe von 3,3',5,5',-Tetramethylbenzidin (TMB)-Lösung und H₂O₂ (Calbiochem) detektiert. Die enzymatische Reaktion wurde mit 1 M HCl gestoppt. Die Messwerte (Mittel± SEM) wurden aus 4 unabhängigen Experimenten, in Triplikaten durchgeführt, ausgewertet und auf unbeschichtetes Titan normalisiert.

### Ergänzende Informationen

### Abspaltung der Peptide von der festen Phase- Details

Folgende scavenger Mischungen wurden genutzt: TIS (Triisopropylsilan)/H₂O (1:1 v/v) für HBP, Thioanisole/ Ethandithiol oder Thioanisole/ Thiocresol (1:1 v/v) für c[RGDfK(Dienophil)] und alle Muschelpeptide, für MP-Dien wurde H₂O genutzt. Die Peptide wurden dann in eiskalten Ethylether gefällt und gewaschen. Das daraus resultierende Pellet wurde gelöst und anschließend lyophilisiert.

Aufreinigung der Peptide wurde auf folgender präparativen RP-HPLC-Säule vorgenommen: Phenomenex Jupiter Proteo C18 Säule (90 Å/ 4µm, 22 mm x 250 mm). Es wurden lineare Gradienten aus Eluent B in Eluent A (A: 0.1 % TFA in H₂O, B: 0.08 % TFA in ACN) genutzt. Die Reinheiten der Peptide wurden mittels folgender Säulen ermittelt: Varian VariTide RPC (200 Å, 6µm), Phenomenex Jupiter Proteo C18 (90 Å/ 4µm) und C18 (300 Å, 5 µm)) mit linearen Gradieneten aus Eluent A und B (A: 0.1 % TFA in H₂O, B: 0.08 % TFA in ACN).

### Analyse der synthetisierten Peptide

**Tabelle 1 Analytik der gereinigten, isolierten Peptide**

| **Peptid** | **Sequenz** | **M [Da]** | **[M+H]⁺** | **Elution (%ACN)^{a}** | **Reinheit [%]** |
|---|---|---|---|---|---|
| **MP** | C-Peg-Y*-K-Y*-Peg-G*- βA-NH2 (SEQ ID NO:8) | 1178.5 | 1179.6 | 33 | >95 |
| **bio-MP** | Bio-X-X-C-Peg-Y*-K- Y*-Peg-G*-βA-NH2 (SEQ ID NO:3) | 1631.0 | 1631.9 | 41 | >95 |
| **bio-(+)mefp-1** | Bio-X-X-A-K-P*-S-Y*- P*-P*-T-Y*-K-NH2 (SEQ ID NO:4) | 1681.7 | 1682.6 | 34 | >95 |
| **bio-(-)mefp-1** | Bio-X-X-A-K-P*-S-Y-P*- P*-T-Y-K-NH2 (SEQ ID NO:5) | 1649.9 | 1651.0 | 33 | >95 |
| **MP-Ahx** | C-X-Y*-K-Y*-X-G*-βA- NH2 (SEQ ID NO:6) | 998.5 | 999.6 | 32 | >95 |
| **RGD** | c[RGDfK(dienophile)] | 915.5 | 916.5 | 44 | >95 |
| **MP-RGD** | C-Peg-Y*-K(c[RGDfK])- Y*-Peg-G*-βA-NH2 | 2349.1 | 2350.1 | 36 ^{b} | >90 |
| **N₃-HBP** | K(N3)-FHRRIKA-NH2 (SEQ ID NO:7) | 1079.7 | 1080.8 | 32 ^{b} | >95 |
| **MP-HBP** | C-Peg-Y*-K-Y*-Peg- G*(K(N₃)-FHRRIKA- NH₂)-βA-NH₂ | 2258.5 | 2259.2 | 32 ^{c} | >95 |
| **MP- RGD-HBP** | C-Peg-Y*-K(c[RGDfK])- Y*-Peg-G*(K(N3)- FHRRIKA-NH2)-βA-NH2 | 3428.8 | 3429.8 | 32 ^{b} | >90 |

Die Identifikation wurde mittels MALDI-ToF MS (Bruker, Daltonics) festgestellt. Die Peptidreinheit wurde mit zwei verschiedenen HPLC-Systemen bestimmt (unterschiedliche Säule und Gradient); ^{a} Phenomenex Jupiter Proteo C18 (300 Å, 5 µm), ^{b} Phenomenex Jupiter Proteo C18 (90 Å, 4 µm) und ^{c} Varian VariTide RPC (200 Å, 6µm)
PEG (Polyethyleneglycol), Y* (L-3,4-Dihydroxyphenylalanin), G* (Propargylglycin), X (Aminohexansäure), Bio (biotin), P* (4-Hydroxyprolin)

### Zell Adhäsion

Um zu testen ob die Zell-abweisenden Eigenschaften durch PEG vermittelt wurden, wurde PEG in MP durch Aminohexansäure ausgetauscht und mit SaOS-2 Zellen wie beschrieben getestet (siehe Figur 6).

### Referenzen

(1) Kumar, V.; Aldrich, J. V. Org Lett 2003, 5, 613.
(2) Prime, K. L.; Whitesides, G. M. J. Am. Chem. Soc. 1993, 115, 10714.
(3) Hassert, R.; Pagel, M.; Ming, Z.; Haupl, T.; Abel, B.; Braun, K.; Wiessler, M.; Beck-Sickinger, A. G. Bioconjug. Chem. 2012, 23, 2129.
(4) Taylor, C. M.; Weir, C. A. J. Org. Chem. 2000, 65, 1414.
(5) Wischerhoff, E.; Uhlig, K.; Lankenau, A.; Borner, H. G.; Laschewsky, A.; Duschl, C.; Lutz, J. F. Angew. Chem. Int. Ed. 2008, 47, 5666.
(6) Geiger, B.; Spatz, J. P.; Bershadsky, A. D. Nat. Rev. Mol. Cell. Bio. 2009, 10, 21.
(7) Pipkorn, R.; Waldeck, W.; Didinger, B.; Koch, M.; Mueller, G.; Wiessler, M.; Braun, K. J. Pept. Sci. 2009, 15, 235.
(8) Böhme, D.; Beck-Sickinger, A. G. Chemmedchem 2015, 10, 804*.*

## Patentansprüche

1. Peptid, umfassend (i) eine Hauptkette umfassend wenigstens ein L-3,4-dihydroxyphenylalanin (DOPA), (ii) wenigstens ein Integrin-Bindepeptid und (iii) wenigstens ein Heparin-Bindepeptid, erhältlich durch eine Kombination von Festphasensynthese und Click-Chemie.

2. Peptid gemäß Anspruch 1, wobei die Hauptkette von einem Protein aus der Muschel *Mytilus edulis* abgeleitet ist.

3. Peptid gemäß Anspruch 1 oder 2, wobei das Peptid auch nicht-peptidische Bestandteile umfassen kann.

4. Peptid gemäß einem der vorherigen Ansprüche, wobei die Hauptkette die Sequenz Cys - PEG - DOPA - Lys - DOPA - PEG - L-Propargylglycin - β-Ala - NH₂ (SEQ ID NO:2) umfasst.

5. Peptid gemäß einem der vorherigen Ansprüche, wobei das Integrin-Bindepeptid die Aminosäuresequenz Arg - Gly - Asp umfasst.

6. Peptid gemäß Anspruch 5, wobei das Integrin-Bindepeptid die Aminosäuresequenz c[Arg - Gly - Asp - D-Phe - Lys] umfasst.

7. Peptid gemäß einem der vorherigen Ansprüche, wobei das Heparin-Bindepeptid die Aminosäuresequenz Phe - His - Arg - Arg - Ile - Lys - Ala (SEQ ID NO:1) umfasst.

8. Beschichtung für Metalloberflächen, umfassend ein Peptid gemäß einem der Ansprüche 1 bis 7.

9. Beschichtung gemäß Anspruch 8, wobei die Metalloberfläche im Wesentlichen aus Titan besteht.

10. Beschichtung gemäß Anspruch 8 oder 9, wobei die Metalloberfläche Teil eines Implantats ist, das zur Implantation in den menschlichen oder tierischen Körper vorgesehen ist, und wobei das Implantat vorzugsweise ausgewählt ist aus der Gruppe bestehend aus Zahnimplantaten und orthopädischen Implantaten.

11. Beschichtete Metalloberfläche, erhältlich durch eine Reaktion von einem Peptid gemäß einem der Ansprüche 1 bis 7 mit einer Metalloberfläche.

12. Beschichtete Metalloberfläche gemäß Anspruch 11, wobei die Metalloberfläche aus Titan besteht.

13. Beschichtete Metalloberfläche gemäß Anspruch 11 oder 12, wobei die Metalloberfläche Teil eines Implantats ist, das zur Implantation in den menschlichen oder tierischen Körper vorgesehen ist, und wobei das Implantat vorzugsweise ausgewählt ist aus der Gruppe bestehend aus Zahnimplantaten und orthopädischen Implantaten.

14. Beschichtetes metallisches Implantat erhältlich durch eine Reaktion von einem Peptid gemäß einem der Ansprüche 1 bis 7 mit einem metallischen Implantat, wobei das metallische Implantat vorzugsweise ausgewählt ist aus der Gruppe bestehend aus Zahnimplantaten und orthopädischen Implantaten.

## Claims

1. A peptide comprising (i) a main chain comprising at least one L-3,4-dihydroxyphenylalanine (DOPA), (ii) at least one integrin binding peptide, and (iii) at least one heparin binding peptide, which can be obtained by a combination of solid phase synthesis and Click chemistry.

2. The peptide according to claim 1, wherein the main chain of a protein is derived from the *Mytilus edulis* cockle.

3. The peptide according to claim 1 or 2, wherein the peptide can also comprise non-peptidic components.

4. The peptide according to any of the preceding claims, wherein the main chain comprises the sequence Cys - PEG - DOPA - Lys - DOPA - PEG - L-propargylglycine - β-Ala - NH₂ (SEQ ID NO:2).

5. The peptide according to any of the preceding claims, wherein the integrin binding peptide comprises the amino acid sequence Arg - Gly - Asp.

6. The peptide according to claim 5, wherein the integrin binding peptide comprises the amino acid sequence c[Arg - Gly - Asp - D-Phe - Lys].

7. The peptide according to any of the preceding claims, wherein the heparin binding peptide comprises the amino acid sequence Phe - His - Arg - Arg - Ile - Lys - Ala (SEQ ID NO:1).

8. A coating for metallic surfaces comprising a peptide according to any of claims 1 to 7.

9. The coating according to claim 8, wherein the metallic surface substantially consists of titanium.

10. The coating according to claim 8 or 9, wherein the metallic surface is part of an implant indented for implantation into the human or animal body; and wherein the implant preferably is selected from the group consisting of dental implants and orthopedic implants.

11. A coated metallic surface which can be obtained by a reaction of a peptide according to any of claims 1 to 7 with a metallic surface.

12. The coated metallic surface according to claim 11, wherein the metallic surface consists of titanium.

13. The coated metallic surface according to claim 11 or 12, wherein the metallic surface is part of an implant indented for implantation into the human or animal body; and wherein the implant preferably is selected from the group consisting of dental implants and orthopedic implants.

14. A coated metallic implant which can be obtained by a reaction of a peptide according to any of claims 1 to 7 with a metallic implant, wherein the metallic implant preferably is selected from the group consisting of dental implants and orthopedic implants.

## Revendications

1. Peptide, comprenant (i) une chaîne principale comprenant au moins une L-3,4-dihydroxyphénylalanine (DOPA), (ii) au moins un peptide de liaison à l'intégrine et (iii) au moins un peptide de liaison à l'héparine pouvant être obtenu par une combinaison de synthèse en phase solide et de chimie clic.

2. Peptide selon la revendication 1, dans lequel la chaîne principale est dérivée d'une protéine de la moule *Mytilus edulis.*

3. Peptide selon la revendication 1 ou 2, dans lequel le peptide peut également comprendre des composants non peptidiques.

4. Peptide selon l'une quelconque des revendications précédentes, dans lequel la chaîne principale comprend la séquence Cys - PEG - DOPA - Lys - DOPA - PEG - L-propargylglycine - β-Ala - NH₂ (SEQ 10 NO:2).

5. Peptide selon l'une quelconque des revendications précédentes, dans lequel le peptide de liaison à l'intégrine comprend la séquence d'acides aminés Arg - Gly - Asp.

6. Peptide selon la revendication 5, dans lequel le peptide de liaison à l'intégrine comprend la séquence d'acides aminés c[Arg - Gly - Asp - D-Phe - Lys].

7. Peptide selon l'une quelconque des revendications précédentes, dans lequel le peptide de liaison à l'héparine comprend la séquence d'acides aminés Phe - His - Arg - Arg - Ile - Lys - Ala (SEQ 10 NO:1).

8. Revêtement pour surfaces métalliques, comprenant un peptide selon l'une quelconque des revendications 1 bis 7.

9. Revêtement selon la revendication 8, dans lequel la surface métallique consiste essentiellement en titane.

10. Revêtement selon la revendication 8 ou 9, dans lequel la surface métallique fait partie d'un implant destiné à être implanté dans le corps humain ou animal, et dans lequel l'implant est de préférence choisi dans le groupe constitué d'implants dentaires et d'implants orthopédiques.

11. Surface métallique revêtue pouvant être obtenue en faisant réagir un peptide selon l'une quelconque des revendications 1 bis 7 avec une surface métallique.

12. Surface métallique revêtue selon la revendication 11, dans lequel la surface métallique est constituée de titane.

13. Surface métallique revêtue selon la revendication 11 ou 12, dans lequel la surface métallique fait partie d'un implant destiné à être implanté dans le corps humain ou animal, et dans lequel l'implant est de préférence choisi dans le groupe constitué d'implants dentaires et d'implants orthopédiques.

14. Implant métallique revêtu pouvant être obtenu en faisant réagir un peptide selon l'une quelconque des revendications 1 bis 7 avec un implant métallique, dans lequel l'implant métallique est de préférence choisi dans le groupe constitué d'implants dentaires et d'implants orthopédiques.
